# EUROPEAN PATENT SPECIFICATION

(11) **EP 1 732 517 B1**
(45) Date of publication and mention of the grant of the patent: **03.05.2017**
(21) Application number: 05717133.2
(22) Date of filing: 23.03.2005
(51) Int. Cl.: A61K 9/20, A61K 31/64

(54) **PROLONGED-RELEASE COMPOSITIONS COMPRISING TORASEMIDE AND A MATRIX-FORMING POLYMER**
RETARDZUSAMMENSETZUNGEN, DIE TORASEMID UND EIN MATRIX BILDENDES POLYMER ENTHALTEN
COMPOSITIONS A LIBERATION PROLONGEE CONTENANT DU TORASEMIDE ET UN POLYMERE DE FORMATION DE MATRICE

(30) Priority: 25.03.2004 ES 200400740
(43) Date of publication of application: 20.12.2006
(73) Proprietor: Ferrer Internacional, S.A., 08028 Barcelona (ES)
(72) Inventor: ROMERO, Alfonso, E-08012 Barcelona (ES); GUERRERO, Marta, E-08024 Barcelona (ES); GUGLIETTA, Antonio, E-08750 Molins de Rei (ES)
(74) Representative: ZBM Patents - Zea, Barlocci & Markvardsen
(86) International application number: PCT/EP2005/051340
(87) International publication number: WO 2005/092291

(56) References cited:
- WO-A-03/035029
- WO-A1-03/074033
- WO-A1-2005/058286
- US-A1- 2003 118 648
- US-A1- 2003 153 608

## Description

### Field of the invention

This invention relates to prolonged-release diuretic compositions containing torasemide as active ingredient.

### Brief description of the drawings

Figure 1 shows the curves of *in-vitro* release rate (cumulative values) of torasemide comparatively for immediate-release (IR) tablets and prolonged-release (PR) tablets according to Example 8.
Figure 2 shows the curves of *in-vitro* release rate of torasemide comparatively for immediate-release (IR) tablets and prolonged-release (PR) tablets according to Example 8.
Figure 3 shows the plasma concentration curves in man after administration of torasemide comparatively for immediate-release (IR) tablets and prolonged-release (PR) tablets according to Example 8.
Figure 4 shows the versus-time curves of the number of urinary urgencies in man after administration of torasemide comparatively for immediate-release (IR) tablets and prolonged-release (PR) tablets according to Example 8.

### Background of the invention

Torasemide (US 4018929) is a potent diuretic with an extensive clinical use. Torasemide mainly acts by inhibiting sodium reabsorption in the ascending limb of Henle's loop (Puschett JB and Jordan LL. Mode of action of Torasemide in man. Progress in Pharmacology and Clinical Pharmacology. 1990;8(1):7-13). Torasemide interferes with Na⁺2Cl⁻K⁺ pump in the luminal cell membrane and blocks the basolateral chloride conductance (Greger R. Inhibition of active NaCl reabsortion in the thick ascending limb of the loop of Henle by torasemide. Arzneim Forsch./Drug Res. 1988;38(1):151-155).

The bioavailability for torasemide is 80-90% after oral administration, the kinetics is linear and the elimination half-life is 3-4 hours. The pharmacokinetic profile is characterized by a peak of maximum plasma concentration (Cₘₐₓ) which is reached within a rather short period of time (tₘₐₓ: approximately 1 hour) and by a rapid elimination (t_{½}: approximately 3-4 hours) (Neugebauer G, Besenfelder E and Möllendorf E. Pharmacokinetics and metabolism of torasemide in man. Arzneim Forsch./Drug Res. 1988;38(1):164-166). Torasemide shows a, linear dose-response relationship at doses, from 2.5 to 20 mg for urinary volume. The sodium excretion exerts a minimal effect on potassium. (Scheen AJ. Dose-response curve of torasemide in healthy volunteers. Arzneim Forsch./Drug Res. 1988;38(1):156-159; Barr WH et al. Torasemide dose-proportionality of pharmacokinetics and pharmacodynamics. Progress in Pharmacology and Clinical Pharmacology. 1990;8(1):29-37). The maximal effects on urine and electrolytes excretions are observed at approximately 2 hours after oral administration (Lesne M. Comparison of the pharmacokinetics and pharmacodynamics of torasemide and furosemide in healthy volunteers. Arzneim Forsch./Drug Res. 1988;38(1):160-163). All these effects clinically become apparent as an acute diuresis and by episodes of urinary urgency and suprapubic discomfort (Lambe R, Kennedy O, Kenny M and Darragh A. Study of tolerance and diuretic properties of torasemide following oral or intravenous administration to healthy volunteers. Eur J Clin Pharmacol 1986;31(Suppl):9-14). US2003/153608, US2003/118648, WO2003/35029, WO2003/74033, WO2005/58286 reveal torasemide containing compositions with varied release pattern. Therefore, the availability of torasemide compositions, which may avoid the troublesome urinary urgencies caused by conventional immediate-release compositions is of a great interest.

### Summary of the invention

An object of the present invention is to prepare diuretic compositions that may provide more stable plasma levels of torasemide in order to avoid the initial peak. This will provide a kinetic profile with fewer fluctuations and steadier levels. Thus, the frequency of urinary urgencies is reduced, which results in a greater comfort for patients who need treatment with torasemide.

The compositions of the present invention comprise torasemide, as active ingredient, and an excipient chosen from matrix-forming polymers, for example, polymers of acrylic acid, cellulose, glycerol behenate, guar gum, xanthan gum, chitosan, gelatin, polyvinyl alcohol and the like. In each composition one only polymer or a mixture thereof may be used. Other components that complete the compositions of the present invention are the usual excipients in pharmaceutical technology comprising diluents, for example, lactose, cellulose, mannitol, calcium phosphate and the like, as well as the mixtures thereof; binding- and disintegrating-action agents, for example, Aerosil^{®} 200, starch, and the like, as well as the mixtures thereof; lubricants, for example, magnesium stearate, talc, and the like, as well as the mixtures thereof. Generally, the compositions of the present invention contain the active ingredient in a proportion from 0.5 to 20%, and the matrix-forming polymer in a proportion from 1 to 40%. according to claim 1. The compositions of the present invention are tablets for oral administration.

The compositions of the present invention maintain diuresis over a maximal period of 24 hours, preferably within the first 12 hours; thus, the possible disturbance of nocturnal enuresis is avoided. As the Cₘₐₓ of plasma levels attained after administration is minimal, the troublesome urinary urgency induced by immediate-release compositions is prevented.

### Detailed description of the invention

The tablets of the present invention contain the active ingredient, torasemide, in an amount of 0.5 to 20 mg. In practice, doses of 5, 10 and 20 mg per tablet are preferred. The matrix-forming polymers are chosen from the following groups: 1) acrylic polymers, for example, Carbopol^{®} (a carbomer -a polymer of acrylic acid polymer), Kollicoat^{®} (a copolymer of methacrylic acid), and their analogues and derivatives; 2) cellulose polymers, for example Methocel^{®} (hydroxypropylmethylcellulose), methylcellulose, sodium carboxymethylcellulose, Natrosol^{®} (hydroxyethylcellulose) and their analogues and derivatives; 3) Compritol^{®} (glyceryl behenate); 4) Meyprogat^{®} (guar gum) and its analogues and derivatives; 5) xanthan gum; 6) chitosan; 7) gelatin; and 8) polyvinyl alcohol and its derivatives.

The compositions of the present invention contain the active ingredient, torasemide, in a proportion from 0.5 to 20% and the matrix-forming polymer in a proportion from 1 to 40%. The most convenient matrix-forming polymer was found to be guar gum, preferably in a proportion of 4%. However, other matrix-forming polymers may be employed in the compositions; their proportions may be varied within a relatively wide range. Thus, Carbopol^{®} is formulated at concentrations from 1 to 20%, preferably 10%, Methocel^{®} at concentrations from 1 to 50%, preferably 40%, Natrosol^{®} and Compritol^{®} at concentrations from 1 to 40%, preferably 20%, Kollicoat^{®} at concentrations from 1 to 40%, preferably 15% and Meyprogat^{®} at concentrations from 1 to 40%, preferably 4%.

The tablets of the present invention are manufactured according to standard procedures of pharmaceutical technology by direct compression or by wet granulation in such a way that moisture of the resulting dry granulate is lower than 10 %.

An *in vitro* dissolution test is performed on the tablets of the present invention using apparatus 2/paddle stirring element (according to U.S. Pharmacopeia) at 50 rpm.

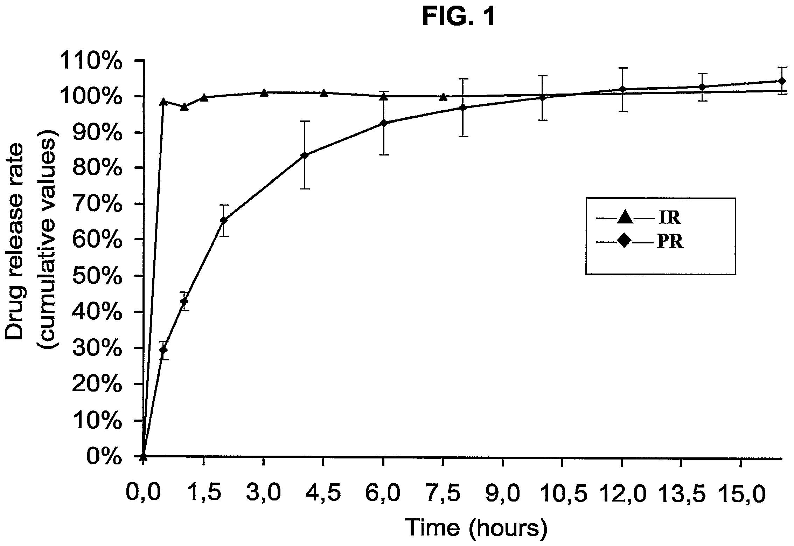

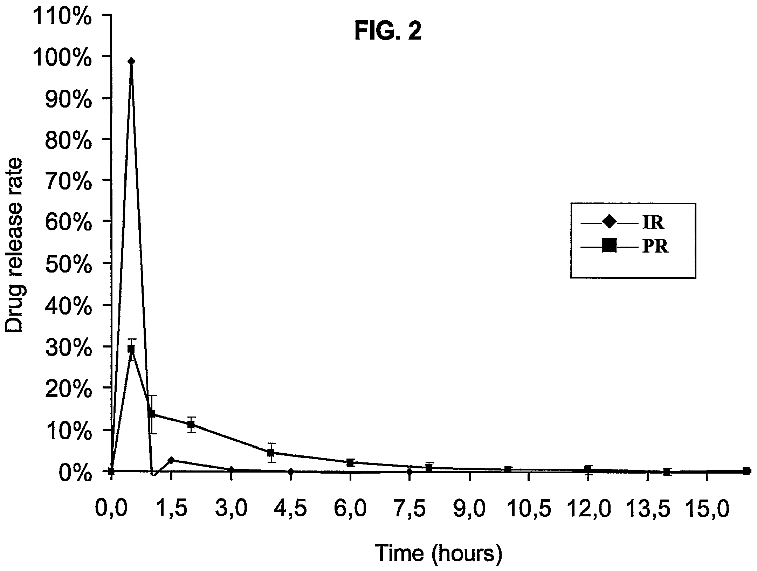

In order to obtain a dissolution profile that fully models the physiological conditions, the test is performed within the first 2 hours at pH 1 and thereafter at pH 6.8. The results obtained are presented in Figures 1 and 2. Fig. 1 shows torasemide release (cumulative values) and Fig. 2 shows torasemide release.

The present invention is further illustrated by - but not limited to - the following examples.

### Example 1: 5 mg tablets of torasemide with Carbopol^{®} and a total weight of 85 mg

| | |
|---|---|
| Torasemide | 5.0 mg |
| Carbopol 940^{®} | 10.0 mg |
| Lactose | 48.0 mg |
| Magnesium stearate | 0.3 mg |
| Aerosil^{®} 200 | 0.5 mg |
| Mannitol q.s. | 85 mg |

### Example 2: 5 mg tablets of torasemide with Methocel^{®} and a total weight of 100 mg

| | |
|---|---|
| Torasemide | 5.0 mg |
| Methocel K 15 M^{®} | 40.0 mg |
| Lactose | 18.0 mg |
| Corn starch | 36.2 mg |
| Pregelatinized starch | 0.3 mg |
| Aerosil^{®} 200 | 0.5 mg |

### Example 3: 5 mg tablets of torasemide with Natrosol^{®} and a total weight of 85 mg

| | |
|---|---|
| Torasemide | 5.0 mg |
| Natrosol HX^{®} | 20.0 mg |
| Magnesium stearate | 0.3 mg |
| Aerosil^{®} 200 | 0.5 mg |
| Microcrystalline cellulose q.s. | 85 mg |

### Example 4: 5 mg tablets of torasemide with Compritol^{®} and a total weight of 100 mg

| | |
|---|---|
| Torasemide | 5.0 mg |
| Compritol 888^{®} | 20.0 mg |
| Lactose | 38.0 mg |
| Corn starch | 36.2 mg |
| Magnesium stearate | 0.3 mg |
| Talc | 0.5 mg |

### Example 5: 10 mg tablets of torasemide with Kollicoat^{®} and a total weight of 85 mg

| | |
|---|---|
| Torasemide | 10.0 mg |
| Kollicoat^{®} SR 30 D | 30.0 mg |
| Magnesium stearate | 0.6 mg |
| Talc | 1.0 mg |
| Calcium phosphate q.s. | 85 mg |

### Example 6: 5 mg tablets of torasemide with Meyprogat^{®} and a total weight of 100 mg

| | |
|---|---|
| Torasemide | 5.0 mg |
| Meyprogat^{®} 90 | 4.0 mg |
| Lactose | 54.0 mg |
| Corn starch | 36.2 mg |
| Magnesium stearate | 0.3 mg |
| Aerosil^{®} 200 | 0.5 mg |

### Example 7: 5 mg tablets of torasemide with Meyprogat^{®} and a total weight of 85 mg

| | |
|---|---|
| Torasemide | 5.0 mg |
| Meyprogat^{®} 90 | 3.4 mg |
| Corn starch | 30.77 mg |
| Aerosil^{®} 200 | 0.42 mg |
| Magnesium stearate | 0.25 mg |
| Lactose | 45.16 mg |

### Example 8: 10 mg tablets of torasemide with Meyprogat^{®} and a total weight of 170 mg

| | |
|---|---|
| Torasemide | 10.0 mg |
| Meyprogat^{®} 90 | 6.8 mg |
| Corn starch | 61.54 mg |
| Aerosil^{®} 200 | 0.85 mg |
| Magnesium stearate | 0.51 mg |
| Lactose | 90.30 mg |

### Example 9: 20 mg tablets of torasemide with Meyprogat^{®} and a total weight of 340 mg

| | |
|---|---|
| Torasemide | 20.0 mg |
| Meyprogat^{®} 90 | 13.6 mg |
| Corn starch | 123.08 mg |
| Aerosil^{®} 200 | 1.70 mg |
| Magnesium stearate | 1.02 mg |
| Lactose | 180.6 mg |

### Example 10: Pharmacokinetics of torasemide in man

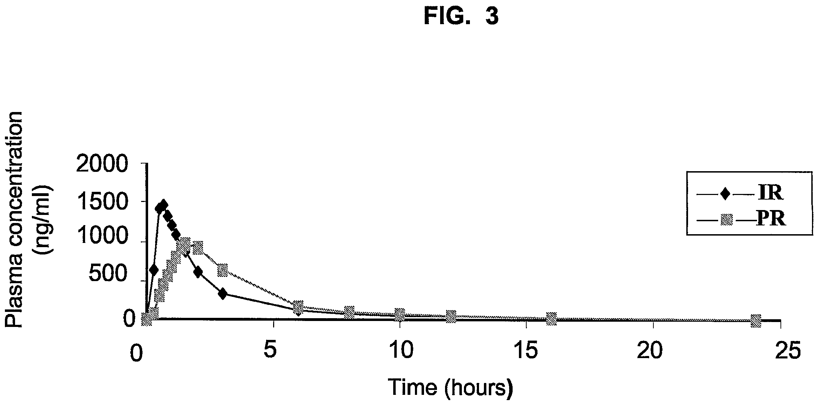

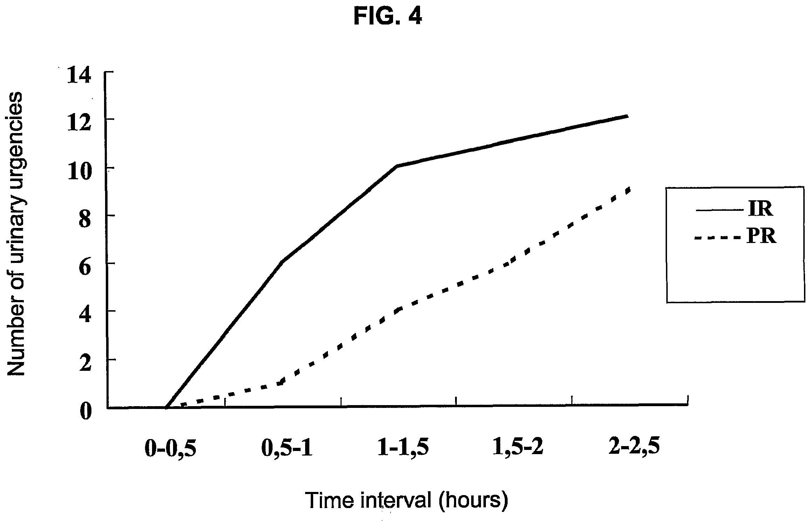

A randomized clinical trial was performed in a group of 10 healthy volunteers who were cross-administered with a 10 mg prolonged-release tablet of torasemide and a 10 mg immediate-release commercial tablet of torasemide (Sutril^{®}, Novag, Spain). There was 1-week interval between the administration of each tablet. The prolonged-release torasemide composition exhibited a lower peak of plasma levels (Cₘₐₓ) attained less acutely (tₘₐₓ) with steadier levels and fewer fluctuations (Fig. 3). The prolonged-release composition produced a lesser frequency of acute diuresis episodes than the immediate-release composition (Fig. 4).

These data show that the compositions of torasemide in the present invention produce a lower peak of plasma levels and fewer fluctuations than the immediate-release composition. In addition, there is a shorter number of urinary urgency episodes after the prolonged-release torasemide composition.

## Claims

1. Prolonged-release compositions containing torasemide as active ingredient, a matrix-forming polymer, and lactose as diluent, wherein:
a) the torasemide is present in a proportion of from 0.5 to 20% by weight; and
b) the matrix-forming polymer is guar gum in a proportion of from 1 to 40 % by weight.

2. Prolonged-release compositions according to claim 1, wherein these compositions are tablets for oral administration.

3. Prolonged-release compositions according to claim 1 and 2, wherein the matrix-forming polymer further comprises one or more of the group of polymers consisting of acrylic acid, cellulose, glycerol behenate, xanthan gum, chitosan, gelatin, polyvinyl alcohol, and mixtures thereof.

4. Prolonged-release compositions according to claim 3, wherein the acrylic matrix-forming polymer is an acrylic acid polymer.

5. Prolonged-release compositions according to claim 3, wherein the cellulose matrix-forming polymer is selected from the group consisting of hydroxypropylmethylcellulose, methylcellulose, carboxymethylcellulose, hydroxyethylcellulose, and their pharmaceutically acceptable salts, and mixtures thereof.

6. Prolonged-release compositions according to any one of claims 1-5, wherein torasemide is present in a proportion from 4 to 10 %.

7. Prolonged-release compositions according to any one of claims 1-6, wherein the matrix-forming polymers are present in a proportion from 2 to 40%.

8. Prolonged-release compositions according to any one of preceding claims comprising in addition one or more excipients selected from the group consisting of diluents, binders, disintegrants, and lubricants.

9. Prolonged-release compositions according to claim 8, wherein, the diluents are selected from the group consisting of celluloses, mannitol, calcium phosphate, and the mixtures thereof.

10. Prolonged-release compositions according to claim 8, wherein, the binders are selected from the group consisting of Aerosil^{®} 200, starch, and mixtures thereof.

11. Prolonged-release compositions according to claim 8, wherein, the disintegrants are selected from the group consisting of Aerosil^{®} 200, starch, and mixtures thereof.

12. Prolonged-release compositions according to claim 8, wherein, the lubricants are selected from the group consisting of magnesium stearate, talc, and mixtures thereof.

## Patentansprüche

1. Retardzusammensetzungen, die Torasemid als Wirkstoff, ein matrixbildendes Polymer und Lactose als Verdünnungsmittel enthalten, wobei:
a) das Torasemid in einem Verhältnis von 0,5 Gew.-% bis 20 Gew.-% vorhanden ist; und
b) das matrixbildende Polymer Guargummi in einem Verhältnis von 1 Gew.-% bis 40 Gew.-% vorhanden ist.

2. Retardzusammensetzungen nach Anspruch 1, wobei die Zusammensetzungen Tabletten zur oralen Verabreichung sind.

3. Retardzusammensetzungen nach Anspruch 1 und 2, wobei das matrixbildende Polymer weiterhin eines oder mehr aus der Gruppe von Polymeren umfasst, die aus Acrylsäure, Cellulose, Glycerolbehenat, Xanthangummi, Chitosan, Gelatine, Polyvinylalkohol und Mischungen davon besteht.

4. Retardzusammensetzungen nach Anspruch 3, wobei das matrixbildende Acrylpolymer ein Acrylsäurepolymer ist.

5. Retardzusammensetzungen nach Anspruch 3, wobei das matrixbildende Cellulosepolymer ausgewählt aus der Gruppe bestehend aus Hydroxypropylmethylcellulose, Methylcellulose, Carboxymethylcellulose, Hydroxyethylcellulose, und deren pharmazeutisch akzeptablen Salzen, und Mischungen davon ist.

6. Retardzusammensetzungen nach einem der Ansprüche 1-5, wobei das Torasemid in einem Verhältnis von 4 bis 10% vorhanden ist.

7. Retardzusammensetzungen nach einem der Ansprüche 1-6, wobei die matrixbildenden Polymere in einem Verhältnis von 2 bis 40% vorhanden sind.

8. Retardzusammensetzungen nach einem der vorhergehenden Ansprüche ferner umfassend einen oder mehrere Hilfsstoffe, die ausgewählt aus der Gruppe bestehend aus Verdünnungsmitteln, Bindemitteln, Zerfallsbeschleunigern und Gleitmitteln sind.

9. Retardzusammensetzungen nach Anspruch 8, wobei die Verdünnungsmittel ausgewählt aus der Gruppe bestehend aus Cellulosen, Mannitol, Calciumphosphat und Mischungen davon sind.

10. Retardzusammensetzungen nach Anspruch 8, wobei die Bindemittel ausgewählt aus der Gruppe bestehend aus Aerosil^{®} 200, Stärke, und Mischungen davon sind.

11. Retardzusammensetzungen nach Anspruch 8, wobei die Zerfallsbeschleuniger ausgewählt aus der Gruppe bestehend aus Aerosil^{®} 200, Stärke, und Mischungen davon sind.

12. Retardzusammensetzungen nach Anspruch 8, wobei die Gleitmittel ausgewählt aus der Gruppe bestehend aus Magnesiumstearat, Talk, und Mischungen davon sind.

## Revendications

1. Compositions à libération prolongée contenant du torasémide comme ingrédient actif, un polymère de formation de matrice, et du lactose comme diluant, dans lesquelles :
a) le torasémide est présent dans un rapport allant de 0,5 à 20% en poids ; et
a) le polymère de formation de matrice est la gomme guar dans un rapport allant de 1 à 40 % en poids.

2. Compositions à libération prolongée selon la revendication 1, dans lesquelles ces compositions sont des tablettes pour l'administration orale.

3. Compositions à libération prolongée selon la revendication 1 et 2, dans lesquelles le polymère de formation de matrice comprend en outre un ou plus dans le groupe de polymères constitué de l'acide acrylique, la cellulose, le béhénate de glycérol, la gomme xanthane, le chitosane, la gélatine, l'alcool polyvinylique, et des mélanges de ceux-ci.

4. Compositions à libération prolongée selon la revendication 3, dans lesquelles le polymère de formation de matrice acrylique est un polymère d'acide acrylique.

5. Compositions à libération prolongée selon la revendication 3, dans lesquelles le polymère de formation de matrice de cellulose est choisi dans le groupe constitué de l'hydroxypropylméthylcellulose, la méthylcellulose, la carboxyméthylcellulose, l'hydroxyéthylcellulose, et leurs sels pharmaceutiquement acceptables, et des mélanges de celles-ci.

6. Compositions à libération prolongée selon l'une quelconque des revendications 1-5, dans lesquelles le torasémide est présent dans un rapport allant de 4 à 10%.

7. Compositions à libération prolongée selon l'une quelconque des revendications 1-6, dans lesquelles les polymères de formation de matrice sont présents dans un rapport allant de 2 à 40%.

8. Compositions à libération prolongée selon l'une quelconque des revendications précédentes comprenant en outre un ou plus excipients choisis dans le groupe constitué des diluants, des liants, des agents de désagrégation, et des lubrifiants.

9. Compositions à libération prolongée selon la revendication 8, dans lesquelles les diluants sont choisis dans le groupe constitué des celluloses, du mannitol, du phosphate de calcium, et des mélanges de ceux-ci.

10. Compositions à libération prolongée selon la revendication 8, dans lesquelles les liants sont choisis dans le groupe constitué de l'Aerosil^{®} 200, de l'amidon, et des mélanges de ceux-ci.

11. Compositions à libération prolongée selon la revendication 8, dans lesquelles les agents de désagrégation sont choisis dans le groupe constitué de l'Aerosil^{®} 200, de l'amidon, et des mélanges de ceux-ci.

12. Compositions à libération prolongée selon la revendication 8, dans lesquelles les lubrifiants sont choisis dans le groupe constitué du stéarate de magnésium, du talc, et des mélanges de ceux-ci.
